# EUROPEAN PATENT APPLICATION

(11) **EP 4 512 420 A1**
(43) Date of publication of application: **26.02.2025**
(21) Application number: 23792262.0
(22) Date of filing: 24.04.2023
(51) Int. Cl.: A61K 39/12, A61K 39/25, A61K 39/20, A61K 38/19, A61P 3/04, A61K 39/00

(54) **COMPOSITION FOR REDUCING SIZE OR VOLUME OF TARGET TISSUE OR KIT INCLUDING SAME**

(30) Priority: 22.04.2022 KR 20220050367
(71) Applicant: SK Bioscience Co., Ltd., Bundang-gu, Seongnam-si, Gyeonggi-do, 13494 (KR)
(72) Inventor: KIM, Eun-som, Seongnam-si, Gyeonggi-do 13494 (KR); SEO, Ki-weon, Seongnam-si, Gyeonggi-do 13494 (KR); HONG, Seung-hye, Seongnam-si, Gyeonggi-do 13494 (KR); KWON, Teawoo, Seongnam-si, Gyeonggi-do 13494 (KR); KIM, Hun, Seongnam-si, Gyeonggi-do 13494 (KR); LEE, Sujeen, Seongnam-si, Gyeonggi-do 13494 (KR); LIM, Seiyoung, Seongnam-si, Gyeonggi-do 13494 (KR)
(74) Representative: Graf von Stosch Patentanwaltsgesellschaft mbH
(86) International application number: PCT/KR2023/005580
(87) International publication number: WO 2023/204693

(57) **Abstract**

The present invention provides a pharmaceutical composition for treating obesity, the composition including: one or more viruses selected from the group consisting of yellow fever virus, herpes zoster virus, and rubella virus; or a genetic material coding for a protein derived from these viruses. Preferably, the pharmaceutical composition is a vaccine composition. The composition provides a reduction in target tissues, preferably tissues containing adipocytes, or an effect that leads to the death of adipocytes.

## Description

### [TECHNICAL FIELD]

The present application claims the priority based on Korean Patent Application No. 10-2022-0050367 filed on April 22, 2022, and the entire contents disclosed in the description and drawings of the corresponding application are incorporated in the present application. The present invention relates to a composition for reducing the size or volume of a target tissue, a kit containing the same, a use thereof, or a method for treating or improving obesity using the same. More particularly, it pertains to a composition that reduces the size or volume of a target tissue by killing mast cells, inhibiting mast cell accumulation, or reducing mast cells. Additionally, it includes a kit containing the same, a use thereof, or a method for treating or improving obesity using the same.

### [BACKGROUND ART]

Obesity refers to a 'condition in which an excessive amount of body fat is accumulated in the body' rather than simply being overweight, and is recognized as a risk factor that increases the incidence of various chronic diseases such as metabolic diseases including diabetes, cardiovascular diseases, cancer and the like. Obesity, defined as excessive accumulation of fat, is accompanied by loss of metabolic, endocrine and immune functions of adipose tissue, and pathological remodeling of this adipose tissue is attracting attention as a pathophysiological factor of major metabolic diseases.

As a drug treatment for treatment of obesity, 'Orlistat', a lipolytic enzyme inhibitor, is used, which plays a role of discharging some of fat in the body out of the body, and side effects include diarrhea and steatorrhea, and the like. In addition, in case of drugs for treatment of obesity, not only do they have many side effects, but also they can decompose fat in an undesired site of a woman's body. At present, there is no appetite suppressant that can be safely used, and in case of severely obese patients with complications, bariatric surgery for the gastrointestinal tract may be helpful.

Meanwhile, while technologies to prevent the formation of fat have been developed, the field of reducing the size of already formed adipocytes and adipose tissue still requires significant research.

### [DISCLOSURE]

### [TECHNICAL PROBLEM]

In order to solve the above problem, the present invention is to provide a new method or pharmaceutical composition which can reduce a size or volume of target tissue by administering into target tissue.

A problem to be solved by the present invention is to provide a new composition for reducing target cells that helps the immune system attack target cells expressing antigen proteins, leading to the apoptosis of the target cells while not affecting other cells in undesired areas.

Additionally, the invention seeks to provide a novel use of the composition that can reduce, decrease, or induce apoptosis in the desired cells or tissues by administering foreign antigens.

### [TECHNICAL SOLUTION]

One embodiment of the present invention provides a composition for the treatment of obesity using a virus or its fragments, an obesity treatment vaccine, a therapeutic method, or a use for treating obesity. One embodiment of the present invention provides a novel pharmaceutical composition (preferably a vaccine composition) for reducing tissue size or volume and a pharmaceutical composition (preferably a vaccine composition) for improving, suppressing or treating obesity, which administers a virus or a genetic material encoding the virus to a target tissue, and when an antigen gene is expressed in a cell constituting the target tissue by the administered genetic material, a pre-existing immunity acts on it to kill the cell. More specifically, the present invention aims to provide a pharmaceutical composition for the prevention or treatment of obesity containing a virus or genetic material coding for the virus, a composition for removing subcutaneous adipocytes, a pharmaceutical composition for reducing the size or volume of tissue (preferably a vaccine composition), and pharmaceutical compositions for improving, inhibiting, or treating obesity. Preferably, the composition may include a vaccine formulation.

The virus of the present invention includes the virus itself, viral-derived proteins, and genetic material coding for viral-derived antigens, and may include fragments, variants, or derivatives thereof. Preferably, the genetic material can be understood to include genetic material coding for viral-derived antigens. In particular, the virus may include one or more selected from the group consisting of yellow fever virus, varicella-zoster virus, and rubella virus. Furthermore, viruses belonging to higher taxa or higher orders that include these viruses may also be included in the present invention, as long as they do not hinder the objectives of the invention.

The inventors of the present invention has confirmed that tissue composed by the cells can be reduced by killing cells constituting a target site using an immune response, thereby completing the present invention. In particular, it has been confirmed through an experiment that the present invention can achieve effective removal and/or reduction of adipocytes, and through this, body shape correction and obesity treatment effects can be shown.

One embodiment of the present invention provides a novel use of a pharmaceutical composition comprising a genetic material, which administers a virus or a composition comprising a virus to a target tissue, and causes an antigen gene to be expressed in cells constituting the target tissue by the administered genetic material, thereby reducing the size of fat, particularly subcutaneous adipocytes. The use provides a use in which, after the composition is administered, a pre-existing immune response acts to kill the corresponding cells, thereby reducing the size or volume of the tissue, and a use in improving, suppressing, or treating obesity.

The inventors of the present invention confirmed that the cells constituting the target area can be killed by using an immune response by a virus, thereby reducing the tissues, and thus completed the present invention. In particular, it was confirmed through experiments that the present invention can effectively remove and/or reduce target cells, thereby exhibiting body shape correction and obesity treatment effects. In particular, when the virus was a yellow fever, shingles, and/or rubella virus, it was advantageous in achieving the effects of the present invention.

One embodiment of the present invention is a pharmaceutical composition for administering to a target tissue to reduce the tissue, wherein the composition may include genetic material encoding a virus, preferably at least one virus selected from the group consisting of yellow fever, shingles, and rubella viruses. One embodiment of the present invention provides a use of the genetic material encoding the virus for target cell killing, target tissue reduction, and more specifically, provides a use of the genetic material for local adipose tissue reduction. Preferably, the genetic material encoding the virus comprises at least one virus selected from the group consisting of yellow fever virus, shingles virus, and rubella virus; or an epitope thereof. One embodiment may provide a use of the virus or an epitope thereof for target cell killing or target tissue reduction. The tissue may be understood as a tissue including adipocytes. The immune action of the present invention may reduce the size of adipocytes or adipose tissue. For example, the adipocytes or adipose tissue may be more preferably subcutaneous adipose tissue. The composition can induce a decrease, reduction or death of adipocytes, particularly adipocytes distributed in subcutaneous adipocytes.

The inventors of the present invention provide a composition or kit that has fewer side effects than existing fat removal surgeries, drugs, etc., and can expect a body shape correction effect by targeting only a local target area (for example, adipose tissue of a specific area). The present invention provides a pharmaceutical composition that acts locally on a target area and reduces the size or volume of a target tissue using immunotherapy. The present invention can preferably provide a pharmaceutical composition, vaccine composition, or therapeutic vaccine composition that induces the death of adipocytes, particularly subcutaneous adipocytes. The present invention can provide a pharmaceutical composition, vaccine composition, or therapeutic vaccine composition that acts locally on a target area and improves, suppresses, or treats obesity using immunotherapy.

The terms "Target tissue or target site" used in the present description means a cell group having a structure and a function for reducing a size or volume. The "figure correction or body shape correction" means having an externally slim and balanced effect by killing or reducing cells constituting tissue (for example, adipose tissue) accumulated in an undesired site to reduce a size or volume of tissue. "Locally act" used in the present description means bring about an effect of cell death or reducing a size or volume of tissue within a radius of 10cm, 9cm, 8cm, 7cm, 6cm, 5cm, 4cm, 3cm, 2cm, 1cm, based on an injection administration site, and preferably, it may bring about the effect within a range of 3cm.

When a subject is exposed to a virus-derived antigen, immunocytes functionally perform functions of phagocytosis and antigen presenting. In one embodiment, the pharmaceutical composition of the present invention may be administered after an immune environment is created by intentionally or unintentionally infiltrating a foreign substance such as a protein antigen or an epitope thereof, or the like to induce an immune response. Through another example, the pharmaceutical composition may be repeatedly administered in the body. In other example, the genetic material comprised in the pharmaceutical composition of the present invention is administered to induce pre-existing immunity, and then with repeated administration of the composition, adipocytes presenting the antigen by a substance secreted by an immune action such as a monocyte, neutrophil, natural killer cell, one kind of lymphocytes, cytotoxic T cell immunocyte, physiologically active substance, antibody, complement, or the like in the body die, and therefore, the size or volume of the adipose tissue may be reduced. The composition may be used as a use of figure correction or body shape correction, and it may be used as a use of body shape correction on a local site. In addition, the composition may be administered to muscle tissue and used as an effect of skin wrinkle improvement such as Botox, a use of figure correction through muscle contraction, and the like. Moreover, it may be used for removing benign or malignant tumors, or removing warts on skin.

The pharmaceutical composition comprises a genetic material encoding one or more a virus-derived antigen, and the genetic material includes not only DNA and/or RNA, but also a viral vector. Also, it comprises antisense nucleotide, mRNA, ssRNA (Single-Stranded RNA), cDNA, and the like, associated with the genetic material. The antigen(s) may also comprise an epitope of an antigen that elicits an immune response.

The inventors of the present invention have confirmed that when a virus-derived antigen, preferably a virus-derived antigen whose association with obesity is unknown, more preferably the genetic material of the virus-derived antigen, and even more preferably the DNA or RNA of the virus-derived antigen, is administered, an excellent effect of reducing the number of adipocytes, reducing the size of adipose tissue, or inhibiting fat accumulation is exhibited. In particular, it has been confirmed that among viruses, yellow fever, shingles, and/or rubella viruses, which were previously unknown to have any association with obesity, have an excellent effect of reducing adipocyte size.

The virus-derived genetic material may be expressed as a viral peptide or a complete protein or a portion thereof. For example, non-limitative examples of the virus include Retroviridae (for example, human immunodeficiency virus, for example, HIV-1; Picornaviridae (for example, poliovirus, hepatitis A virus; enterovirus, human coxsackie virus, rhinovirus, echovirus); Calciviridae (for example, strain causing gastroenteritis); Togaviridae (for example, equine encephalitis virus, rubella virus); Flaviridae (for example, dengue virus, encephalitis virus, yellow fever virus); Coronoviridae (for example, corona virus); Rhabdoviridae (for example, vesicular stomatitis virus, rabies virus); Filoviridae (for example, Ebola virus); Paramyxoviridae (for example, parainfluenza virus, mumps virus, measles virus, respiratory syncytial virus); Orthomyxoviridae (for example, influenza virus); Bungaviridae (for example, Hantaan virus, Bunga virus, and phlebovirus and Nairo virus); Arena viridae (hemorrhagic fever virus); Reoviridae (for example, reovirus, orbivirus and rotavirus); Birnaviridae; Hepadnaviridae (hepatitis B virus); Parvoviridae (parvovirus); Papovaviridae (papillomavirus, polyomavirus); Herpesviridae (herpes simplex virus (HSV) 1 and 2, varicella-zoster virus, cytomegalovirus (CMV), herpes virus; Poxviridae (variola virus, vaccinia virus, poxvirus); and Iridoviridae (for example, African swine fever virus). Preferably, it may comprise Rubivirus genus, more preferably Rubella virus (also called rubella virus); preferably Flavivirus genus, more preferably Yellow fever virus; preferably Varicellovirus genus, more preferably varicella-zoster virus. When the virus was administered, the effect of reducing tissue size was excellent. In addition, when the above virus was administered, there were few side effects and it was safe. The virus was easy to formulate. Even if a small amount of the virus is administered, it can stimulate pre-existing immunity, thereby quickly bringing about a fat decomposition effect.

Preferably, the genetic material encoding the virus may comprise a nucleic acid molecule having a nucleic acid sequence consisting of a polynucleotide selected from the group consisting of SEQ ID NOs: 1 to 6. Alternatively, the genetic material may comprise a nucleic acid molecule encoding any one or more peptides selected from the group consisting of SEQ ID NOs: 12 to 14.

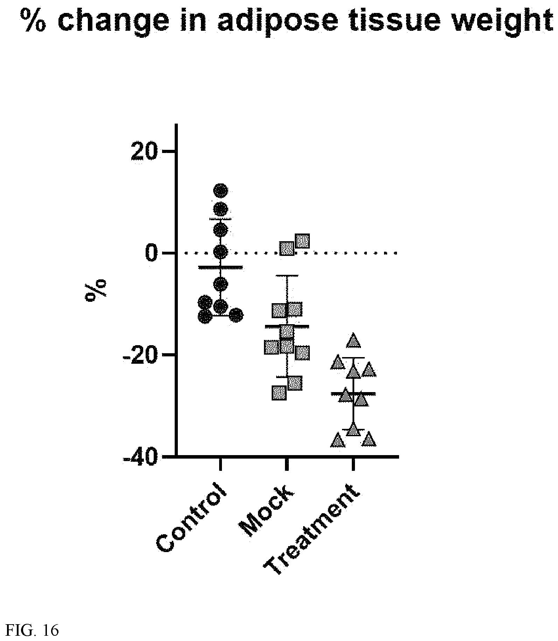

One embodiment of the present invention can provide a pharmaceutical composition, a vaccine composition, or a therapeutic vaccine composition comprising a nucleic acid molecule comprising a polynucleotide selected from the group consisting of SEQ ID NO: 2, SEQ ID NO: 4, and SEQ ID NO: 6. Wherein SEQ ID NO: 2, SEQ ID NO: 4, and SEQ ID NO: 6 can include a polynucleotide sequence encoding at least one virus (or a protein derived from a virus) selected from the group consisting of yellow fever virus, varicella-zoster virus, and rubella virus following a capping sequence, a 5'-UTR, a Kozak sequence. A 3'-UTR sequence, a poly A Tail, and a primer can be linked following the polynucleotide sequence encoding the virus (or a protein derived from a virus), unless otherwise specified, and these are connected in the 5' to 3' direction. See FIGS. 16 to 18 .

In addition, the genetic material may include a nucleic acid sequence consisting of a polynucleotide selected from the group consisting of SEQ ID NOs: 1 to 6; or a polynucleotide having a nucleic acid sequence having at least 85% sequence homology with the nucleic acid sequence and capable of reducing target tissue size or volume, decomposing fat, or treating, improving, or preventing obesity (i.e., the present invention can achieve the purpose). The sequence homology may include a sequence homology of 85% or more, 90% or more, 95% or more, or 99% or more. Or, the genetic material may include a polynucleotide having a nucleic acid sequence having at least 85% sequence homology with a nucleic acid molecule encoding any one or more peptides selected from the group consisting of SEQ ID NOs: 12 to 14 and capable of reducing target tissue size or volume (i.e., the present invention can achieve the purpose). The sequence homology may include a sequence homology of 85% or more, 90% or more, 95% or more, 99% or more, or 100%.

The composition may further comprise a genetic material encoding at least one cytokine selected from the group consisting of IL-12, IL-2, IL-4, IL-5, IFN-γ, IL-10, IL-1, IL-6, INF-alpha, INF-beta, TNF-alpha, and TNF-beta, and it is preferable to comprise IL-12 to further maximize the immunostimulatory effect by an antigen expressed by the genetic material. For example, the IL-12 may comprise a DNA sequence consisting of SEQ ID NO: 7 and/or SEQ ID NO: 8.

In a preferred embodiment, the viral genetic material can be included in a plasmid (e.g., gWizTM vector) that enables the expression of a gene corresponding to a structural protein of the virus in an animal cell.

In a preferred embodiment, the virus can include a yellow fever virus, for example, but not limited to, the yellow fever virus can include 17D strain. The plasmid can include a nucleic acid molecule encoding the virus, or a fragment, variant, or analog thereof. For example, it can include SEQ ID NO: 1 or SEQ ID NO: 2; or a fragment, variant, or analog thereof.

The nucleic acid molecule encoding the virus may comprise a nucleic acid molecule encoding a structural protein. For example, it may comprise a nucleic acid molecule encoding a protein of SEQ ID NO: 12, or a fragment, variant or analog thereof.

In a preferred embodiment, the viral genetic material can be included in a plasmid (e.g., gWizTM vector) that enables protein expression in an animal cell of a gene corresponding to a structural protein of a Rubella virus. The Rubella virus can be used without limitation, and for example, the Rubella virus can include, but is not limited to, RA27/3 strain. The gene encoding the virus can include a gene encoding spike glycoproteins. The plasmid can include a nucleic acid molecule encoding the virus or a fragment, variant, or analog thereof. For example, it can include SEQ ID NO: 5 or SEQ ID NO: 6; or a fragment, variant, or analog thereof.

Examples of the spike glycoproteins may include E2-E1 heterozygotes. For example, the nucleic acid molecule encoding the protein of SEQ ID NO: 14 or a fragment, variant or analog thereof may be included.

In one preferable embodiment, the composition may comprise a gene corresponding to a structural protein of a rubella virus gene in a plasmid enabling protein expression in an animal cell (for example, gWiz^{™} vector). The rubella virus may be used without limitation, and for example, the rubella virus may include RA27/3 strain, but not limited thereto. The gene encoding the virus may comprise a gene encoding spike glycoproteins. The example of the spike glycoproteins may include E2-E1 heterozygotes. For example, it may comprise a nucleic acid molecule encoding protein of SEQ ID NO: 5 or a fragment, variant or analogue thereof.

In one preferable embodiment, the composition may comprise a gene corresponding to a structural protein of a varicella-zoster virus gene in a plasmid enabling protein expression in an animal cell (for example, gWiz^{™} vector). The varicella-zoster virus may be used without limitation, and for example, the varicella-zoster virus may include Oka strain, but not limited thereto. The above plasmid may comprise a nucleic acid molecule encoding a virus or a fragment, variant, or analog thereof. For example, it may comprise SEQ ID NO: 3 or SEQ ID NO: 4; or a fragment, variant, or analog thereof.

The nucleic acid molecule encoding the virus may comprise a nucleic acid virus encoding gE protein, a surface protein. For example, it may comprise a nucleic acid molecule encoding protein of SEQ ID NO: 13 or a fragment, variant or analogue thereof.

Here, the term 'fragment, variant or analog thereof may be understood understood as meaning a part of a proteins, peptides, nucleic acids, nucleic acid molecules or genes which achieves the purpose intended by the proteins, peptides, nucleic acids, nucleic acid molecules or genes of the present invention, but may have a sequence homology of 85% or more, 90% or more, 95% or more or 99% or more. For example, the meaning of comprising the fragment, variant or analogue may be understood as meaning that if it includes a nucleic acid molecule composed of any one or more polynucleotides selected from the group consisting of SEQ ID NOs: 1 to 6, it is not excluded from the scope of the present invention.

The virus may have an excellent effect of death of target tissue, preferably, adipocytes, and reducing a size of tissue. In addition, the effect may be quickly exhibited, and there are few side effects. Moreover, there may be little risk of deterioration even if stored for a long time as an injection.

In other embodiment, the composition may further enhance an effect of adipocyte death, or reducing target tissue, by comprising an immune enhancer capable of enhancing a cellular immune response. The composition may further comprise a genetic material encoding an immune enhancer capable of improving an immune enhancing effect, for example, an auxiliary genetic material encoding interleukin 12 (IL-12). The auxiliary genetic material may be provided by inserting a coding region for example, into pSF-CMV-CMV-Sbfl vector.

The composition of the present invention may be used with not only the cytokine, a gene encoding the same or other immunoadjuvant, but also a pharmaceutical carrier or excipient as widely used in the art. The composition may be formulated for human or veterinary use, and administered in various routes. As an administration route, an oral, transdermal, intramuscular, peritoneal, intravenous, subcutaneous, or intranasal route may be used, but not limited thereto. More preferably, a transdermal, intramuscular, peritoneal or subcutaneous route may be used. In addition, the composition may be administered by any device or route in which an active material can move to a target cell, and the administration method is not particularly limited, as long as the composition of the present invention or the genetic material comprised in the composition can be delivered to target tissue or a cell constituting the tissue. For example, it may be formulated and provided as an injection, microneedle patch, or the like. For example, when used as an injection, the injection may be prepared using an aqueous solvent such as physiological saline solution, Ringer solution and the like, a non-aqueous solvent such as plant oil, higher fatty acid ester (e.g., oleic acid ethyl, etc.), alcohols (e.g., ethanol, benzyl alcohol, propylene glycol, glycerin, etc.) and the like, within a range that does not impede the effect of the genetic material, and may comprise a pharmaceutical carrier such as a stabilizer for preventing deterioration (e.g., ascorbic acid, sodium hydrogen sulfite, sodium pyrosulfite, BHA, tocopherol, EDTA, etc.), an emulsifier, a buffer for adjusting pH, a preservative for preventing microbial growth (e.g., phenyl mercury nitrate, thimerosal, benzalkonium chloride, phenol, cresol, benzyl alcohol, etc.), and the like. When used as a microneedle patch, the microneedle may include both insoluble and soluble microneedles.

In addition, the genetic material and/or auxiliary genetic material comprised in the composition may be delivered into the body according to a common gene delivery method in the art. As a non-limitative example, gene delivery methods such as physical delivery methods such as electroporation or gene gun and chemical delivery methods such as lipid-gene complex (Lipid-DNA complex: Lipoplex), polymer-gene complex (Polymer-DNA complex: Polyplex), liposome, dendrimers, nanoparticles, or other appropriate transfer vectors may all be used.

The composition of the present invention is administered in a pharmaceutically effective dose. The term "pharmaceutically effective dose" refers to an amount sufficient for a composition to exhibit an effect of treatment, improvement or prevention, or to exhibit a vaccine effect, and in addition, it means an amount sufficient to not cause side effects or a severe or excessive immune response. The composition of the present invention may be administered with a genetic material due to characteristics of the purpose of inducing an immune response and inducing cell death, and it is preferable to administer it 4-8 times, preferably, 5 times at a 2-3 day interval. When administered as described above, an effect of decrease or reduction of tissue may be obtained by cell death of the target site. The composition may have a difference in the cell death effect as it is administered in large amounts or multiple times, and may be used by adjusting an appropriate dosage for each site.

Other embodiment of the present invention provides a method for reducing or decreasing a size of volume of tissue, comprising administering a genetic material encoding a genetic material encoding an antigen, preferably a virus, such as yellow fever, shingles or rubella virus, a vector comprising the genetic material, or a composition comprising thereof into target tissue of a subject. The present method may be effective in killing cells that make up subcutaneous adipocyte tissue, reducing fat weight, or reducing cell size. The i) method; or ii) the genetic material encoding a virus or virus-derived protein, a vector comprising the genetic material, or a composition comprising thereof, or a kit comprising thereof may be used as a use for improving or treating obesity of a subject, a use for wrinkle improvement, a use for muscle reduction, a use for removing or reducing benign or malignant tumors, a use for removing warts, and the like, and may be used as a use for removing fat pockets under eyes for a cosmetic purpose, and may be used as a use for alleviating dark circles through this.

The method may further comprise composing an immune environment in the body prior to administering the genetic material, a vector comprising the genetic material, or a composition comprising thereof into target issue. The composing an immune environment in the body may comprise administering a foreign substance capable of inducing an immune response. The foreign substance may include any substance as long as it uses protein as an antigen without limitation.

One embodiment of the present invention may be provided as a use for treatment, improvement or prevention of various indications, and may be provided as a cosmetic purpose for improvement of appearance, as well.

One embodiment of the present invention can be provided as a vaccine for treating obesity. The therapeutic vaccine can be defined as a vaccine in that it utilizes an immune response in the body, and can be defined as a therapeutic vaccine in that it affects fat, adipocytes, or adipose tissues that have already been created to reduce their size or kill cells. Therefore, the vaccine for treating obesity can be understood as a concept that is distinct from a preventive vaccine that simply suppresses the formation of adipocytes or suppresses the accumulation of fat.

Other example of the present invention provides a kit, comprising (a) a vaccine composition for reducing the size or volume of a target tissue, comprising at least one virus selected from the group consisting of yellow fever, shingles, and rubella viruses; a protein derived from said virus; or genetic material encoding said virus or protein, preferably comprising at least one virus selected from the group consisting of yellow fever, shingles, and rubella viruses or genetic material encoding said virus or protein, and (b) an administration guide for administration of the vaccine. The kit may further include (c) a composition comprising a protein antigen or an epitope thereof for immune environment composition prior to administering the (a) composition. Herein, the (c) may be interpreted as at least one pre-vaccine or composition for inducing an immune response.

The 'pre-vaccine' mentioned herein can be understood as a vaccine composition administered before the 'pharmaceutical composition for improving, suppressing, or treating obesity' of the present invention, or the 'composition for reducing target tissue size or volume' is administered to obtain pre-existing immunity. The pre-vaccine may include an antigenic substance identical to the antigenic substance included in the 'pharmaceutical composition for improving, suppressing, or treating obesity' of the present invention, or the 'composition for reducing target tissue size or volume', but is not limited thereto. For example, (a) the 'pharmaceutical composition for improving, suppressing, or treating obesity', or the 'composition for reducing target tissue size or volume', comprising genetic material encoding a virus, preferably a yellow fever, shingles, and/or rubella virus, may include at least one virus selected from the group consisting of yellow fever, shingles, and rubella viruses, and (c) may also include at least one virus selected from the group consisting of yellow fever, shingles, and rubella viruses. As another example, (a) a 'pharmaceutical composition for improving, suppressing, or treating obesity', or a 'composition for reducing target tissue size or volume', comprising genetic material encoding a virus, preferably at least one virus selected from the group consisting of yellow fever, shingles, and rubella viruses, may comprise at least one virus selected from the group consisting of yellow fever, shingles, and rubella viruses, and (c) may comprise a vaccine that is administered in infants to induce the retention of antibodies in the body.

The composition (a) above may further include a genetic material encoding a cytokine, and a material that can be used to increase the immune effect of the composition (a) above may be included without limitation. The genetic material encoding the cytokine may be replaced with a protein expressed by the genetic material. The cytokine may include at least one selected from the group consisting of IL-12, IL-2, IL-4, IL-5, IFN-γ, IL-10, IL-1, IL-6, INF-alpha, INF-beta, TNF-alpha, and TNF-beta, and in addition to the cytokines above, cytokines known in the art may be included without limitation.

The kit of the present invention, may comprise at least one additional agent as defined in the present description in relation to a pharmaceutical composition, an antimicrobial agent, a DNase inhibitor, a RNase inhibitor, a solubilizing agent, and the like. The kit may for example, consist of at least one kit parts (for example, a container). For example, each container may be a syringe, a prefilled syringe, vial, bottle, jar, sealed sleeve, envelope or pouch, tube or blister package or any other suitable form in which the container is configured to prevent premature mixing of elements. Each of different elements may be provided individually, or some of the different elements may be provided together (i.e., in the same container). The kit may also comprise an administration guide having information on administration, administration method and dosage of any component.

Another embodiment of the present invention provides a syringe filled with a composition for improving, suppressing, or treating obesity, preferably a vaccine composition, comprising genetic material encoding a virus or a viral protein. Another embodiment of the present invention provides a syringe filled with a pharmaceutical composition for reducing target tissue size or volume, preferably a vaccine composition, comprising genetic material encoding a virus or a viral protein. Preferably, a syringe filled with a composition for improving, suppressing, or treating obesity, preferably a vaccine composition, comprising at least one virus selected from the group consisting of yellow fever, varicella-zoster virus, and rubella viruses; or a genetic material encoding the virus-derived protein; or a vaccine composition for reducing target tissue size or volume. The syringe may further contain a genetic material encoding a cytokine. The cytokine may include at least one selected from the group consisting of IL-12, IL-2, IL-4, IL-5, IFN-γ, IL-10, IL-1, IL-6, INF-alpha, INF-beta, TNF-alpha, and TNF-beta.

One embodiment of the present invention is to provide a pharmaceutical composition for improving, suppressing, or treating obesity, comprising genetic material encoding at least one virus selected from the group consisting of yellow fever, shingles, and rubella viruses; or a protein derived from said virus.

The pharmaceutical composition can reduce the size or volume of a target tissue.

The genetic material may be a polynucleotide encoding at least one protein selected from the group consisting of a structural protein of yellow fever virus, glycoprotein E of shingles virus, and E2 and E1 proteins of rubella virus.

The polynucleotide may be DNA or RNA, and preferably may be DNA or mRNA.

The genetic material may comprise a nucleic acid molecule composed of one or more polynucleotides selected from the group consisting of SEQ ID NOS: 1 to 6.

The pharmaceutical composition may further comprise a genetic material encoding a cytokine.

The cytokine may be one or more selected from the group consisting of IL-12, IL-2, IL-4, IL-5, IFN-γ, IL-10, IL-1, IL-6, INF-alpha, INF-beta, TNF-alpha, and TNF-beta. For example, the genetic material may comprise a polynucleotide DNA sequence represented by SEQ ID NOS: 7 and 8, respectively.

The genetic material may induce an intracellular immune response.

The composition acts locally, and the composition may be applied after at least one antigen for creating an immune environment is first applied to the subject.

The antigen may include a virus, protein, or an epitope thereof introduced from outside the human body. Preferably, the genetic material may be administered to the target tissue by oral administration, transdermal, intramuscular, peritoneal, intravenous, subcutaneous, or nasal injection, or by electroporation, gene gun, liposome, dendrimers, nanoparticles, or transfer vector.

The dosage of the genetic material included in the composition may be 0.1 to 1000 ug/site per single inoculation.

The composition is administered to an individual for the purpose of reducing or shrinking the size of adipose tissue, or killing it, and the composition may be administered at least once to a site for the purpose of reducing or shrinking the size of adipose tissue, or killing it after confirming the production of antibodies against at least one of the viruses after administration of a vaccine for preventing any one or more viruses selected from the group consisting of yellow fever, shingles, and rubella viruses.

The composition may be provided as a vaccine, such as a therapeutic agent, a therapeutic vaccine, or a prophylactic vaccine.

Another embodiment may provide a kit for providing the composition. The kit may include, in addition to the composition, an administration guideline for administering the composition.

The kit may further include an additional composition comprising an antigen for creating an immune environment in the body before administering the composition. The kit may further include a genetic material encoding a cytokine.

Another embodiment may provide a pharmaceutical composition for reducing the size or volume of a target tissue, comprising at least one virus selected from the group consisting of yellow fever, varicella-zoster virus, and rubella viruses; or a genetic material encoding a protein derived from the virus.

The pharmaceutical composition may reduce the number of adipocytes or the size of adipocyte tissue, or induce death of the cell or tissue.

According to one embodiment, the present invention provides a method for reducing the size or volume of a target tissue by administering the composition to a subject in need thereof. Another embodiment provides a method of suppressing, improving, or treating obesity by administering the composition to a subject in need thereof. The subject may include, without limitation, a mammal, such as a dog, a cow, a pig, a horse, a human, and the like, and preferably a human.

In one embodiment, the method may provide a method for improving, suppressing, or treating obesity, or a method for reducing, shrinking, or killing adipose tissue, in a subject for the purpose of reducing, shrinking, or killing adipose tissue, comprising the steps of: i) administering a vaccine for preventing at least one virus selected from the group consisting of yellow fever, shingles, and rubella viruses, ii) confirming the production of antibodies by the vaccine, and iii) administering a pharmaceutical composition comprising at least one virus selected from the group consisting of yellow fever, shingles, and rubella viruses; a protein derived from the virus; or a genetic material encoding the virus or protein. The above method can repeat step iii) 1 or more times, 2 or more times, 3 or more times, 4 or more times, 5 or more times, 6 or more times, 7 or more times, 8 or more times, 9 or more times, or 10 or more times, preferably 2 or more times, 3 or more times, 4 or more times, 5 or more times, 6 or more times, 7 or more times, 8 or more times, 9 or more times, or 10 or more times, more preferably 3 or more times, 4 or more times, 5 or more times, 6 or more times, 7 or more times, 8 or more times, 9 or more times, or 10 or more times. Preferably, step iii) can be repeated at intervals of 1 to 10 days, at intervals of 2 to 8 days, or at intervals of 3 to 7 days.

The composition used in step iii) above may further comprise one or more cytokines, preferably the cytokines may comprise one or more cytokines selected from the group consisting of IL-12, IL-2, IL-4, IL-5, IFN-γ, IL-10, IL-1, IL-6, INF-alpha, INF-beta, TNF-alpha, and TNF-beta. As another alternative, the step iii) above may comprise a step of iv) administering a composition comprising one or more cytokines, preferably the cytokines may comprise one or more cytokines selected from the group consisting of IL-12, IL-2, IL-4, IL-5, IFN-γ, IL-10, IL-1, IL-6, INF-alpha, INF-beta, TNF-alpha, and TNF-beta. That is, the cytokine may be administered together with or sequentially with a composition containing the genetic material of the virus or virus-derived protein of the present invention.

One embodiment of the present invention provides a method of improving or therapeutically improving a portion of a virus selected from the group consisting of yellow fever, shingles, and rubella viruses; the viral protein; or a substance encoding the virus. Another example provides for the use of a composition comprising at least one virus selected from the group consisting of yellow fever, varicella-zoster virus, and rubella viruses; a protein derived from said virus; or genetic material encoding said virus or protein, for reducing the size or volume of a target tissue.

The above use may be used for the purpose of reducing the size or volume of a target tissue. Preferably, the target tissue may comprise a tissue comprising adipocyte cells.

The genetic material may be a polynucleotide encoding at least one protein selected from the group consisting of a structural protein of a yellow fever virus, a glycoprotein E of a varicella-zoster virus, and E2 and E1 proteins of a rubella virus.

The poly polygon may comprise DNA or RNA, and the RNA may alternatively comprise mRNA.

The genetic material may comprise a nucleic acid molecule consisting of one or more polynucleotides selected from the group consisting of SEQ ID NOS: 1 to 6.

The genetic material may be used to induce an immune response within a cell.

The composition may be administered to act locally, and the composition may be applied after at least one antigen for creating an immune environment has been first applied to the subject.

In one embodiment, the composition is administered to a subject for reducing the size, shrinking the size, or killing adipose tissue, and the composition may be administered at least once to an area for the purpose of reducing the size, shrinking the size, or killing adipose tissue after administration of a vaccine for preventing any one or more viruses selected from the group consisting of yellow fever, shingles, and rubella viruses and confirmation of antibody production against any one or more of the viruses.

### [ADVANTAGEOUS EFFECTS]

The present invention provides the effects of reducing the size or volume of a tissue in a specific targeted area. The present invention offers a new type of composition or therapeutic vaccine that can improve or treat obesity. This composition has the ability to destroy cells in a specific target tissue, leading to a reduction in the size or volume of that tissue. By administering the composition, a body shaping effect or a body improvement effect can be obtained.

The present invention can provide a new type of obesity treatment which can solve local obesity and complete a smooth body line.

The composition for improving, suppressing, or treating obesity in the present invention is effective for selective local site reduction, allowing for the selective reduction of tissue only in the desired area, without affecting tissue in undesired areas or the cells that constitute them.

In addition, the composition of the present invention can be used for a cosmetic purpose for appearance improvement.

### [BRIEF DESCRIPTION OF THE DRAWINGS]

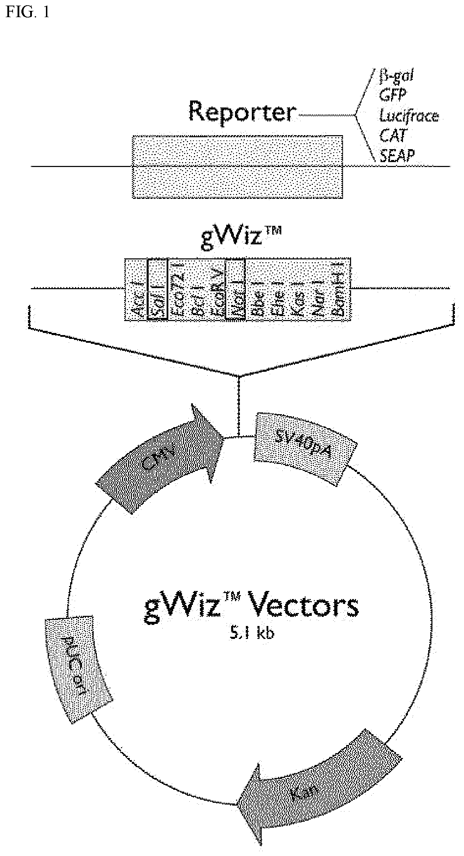

FIG. 1 shows the vector map of the gWizTM vector for inserting the genetic material of the present invention. The gene was inserted at the restriction enzyme sites SalI and NotI.

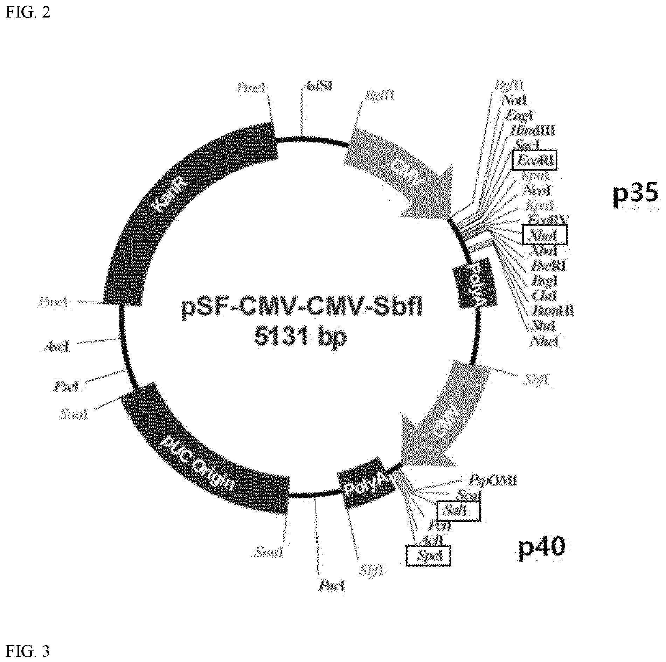

FIG.2 shows the vector map of the pSF-CMV-CMV-Sbfl vector, which was used to insert the nucleic acid sequences corresponding to the coding regions of IL-12 p35 (GenBank: M86672.1) and p40 (GenBank: M86671.1) (p35; 127-774 bp, p40; 35-1042 bp). IL-12 p35 was inserted at the restriction enzyme sites EcoRI and XhoI in MCS site I, while IL-12 p40 was inserted at the restriction enzyme sites SalI and SpeI in MCS site II.
FIG.3 shows the vector for in vitro transcription (IVT).

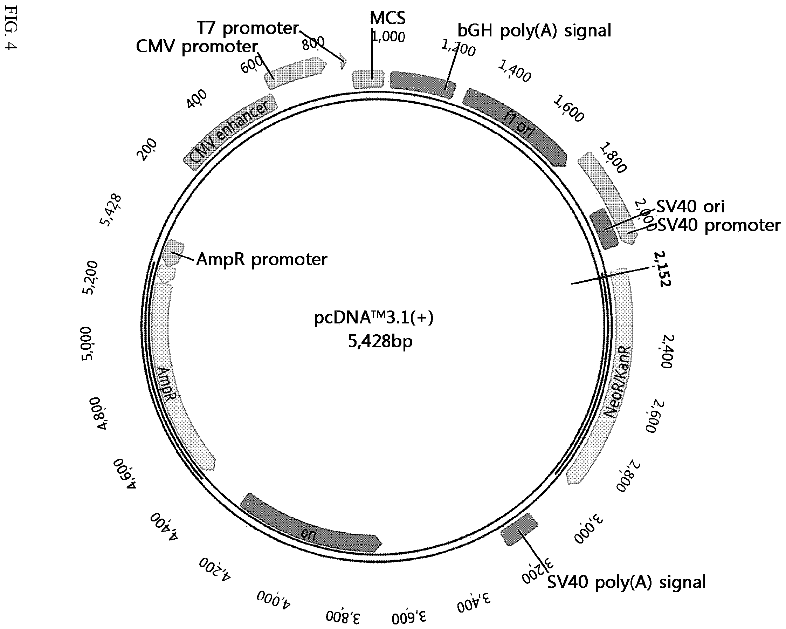

FIG.4 presents the results of inducing pre-existing immunity by administering a YFV vaccine strain (live attenuated virus) to mice. After administering the vaccine strain three times at two-week intervals, the levels of YFV-specific IgG antibodies in the blood were measured two weeks after the last administration.
   NC (negative control) indicates normal mice that received only the medium, YFV-ND indicates YFV-immunized normal mice fed a normal diet, and YFV-HFD indicates YFV-immunized obese mice fed a high-fat diet.

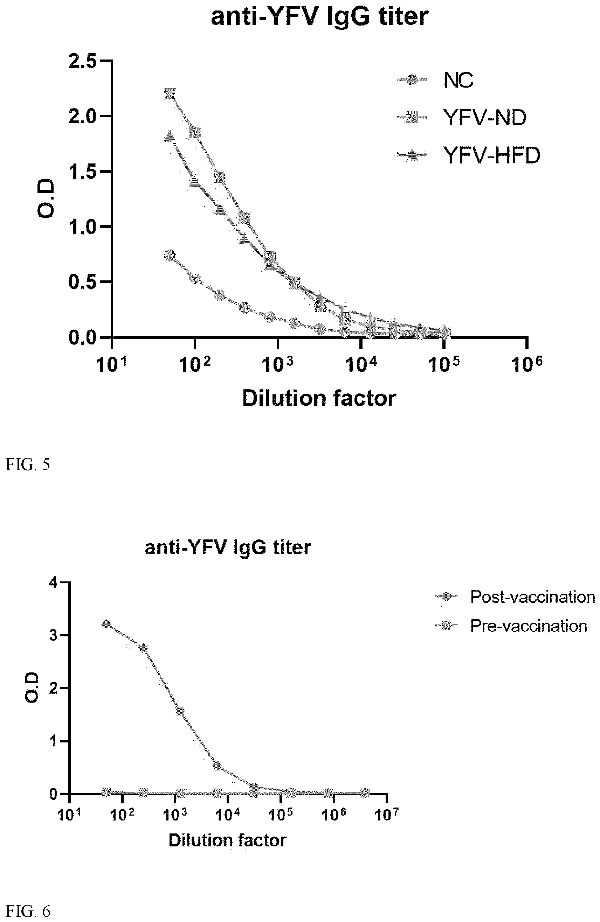

FIG. 5 also shows the results of inducing pre-existing immunity by administering a YFV vaccine strain (live attenuated virus) to mice. After administering the vaccine strain twice at two-day intervals, YFV-specific IgG antibody levels in the blood were measured three weeks later. Pre-vaccination refers to some individuals before YFV administration, while post-vaccination refers to YFV-immunized obese mice fed a high-fat diet.

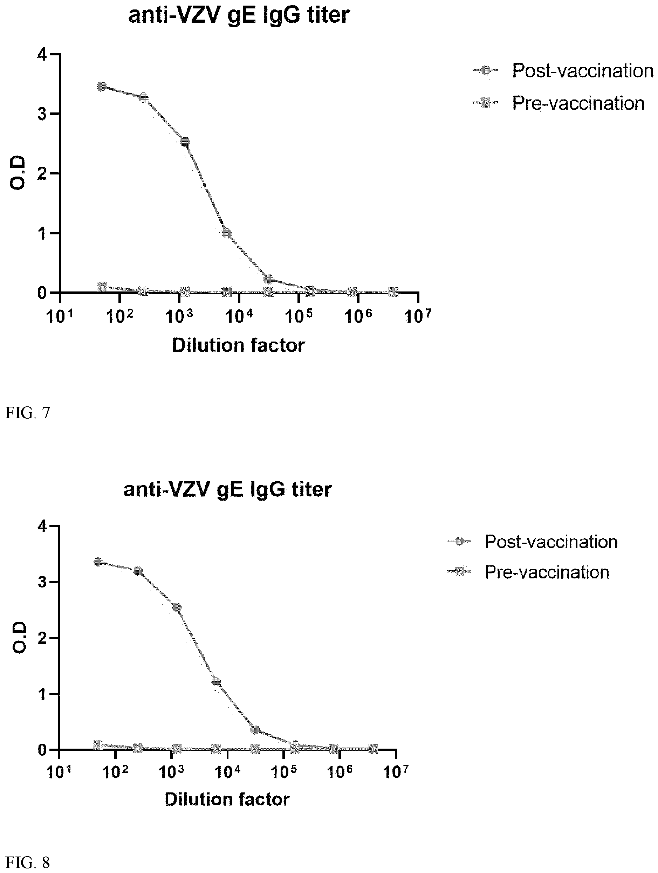

FIG.6 and FIG.7 show the results of inducing pre-existing immunity by administering a VZV vaccine strain (live attenuated virus) to mice. After administering the vaccine strain twice at four-week intervals, VZV gE protein-specific IgG antibody levels in the blood were measured three weeks later. Pre-vaccination refers to some individuals before VZV administration, while post-vaccination refers to VZV-immunized obese mice fed a high-fat diet.

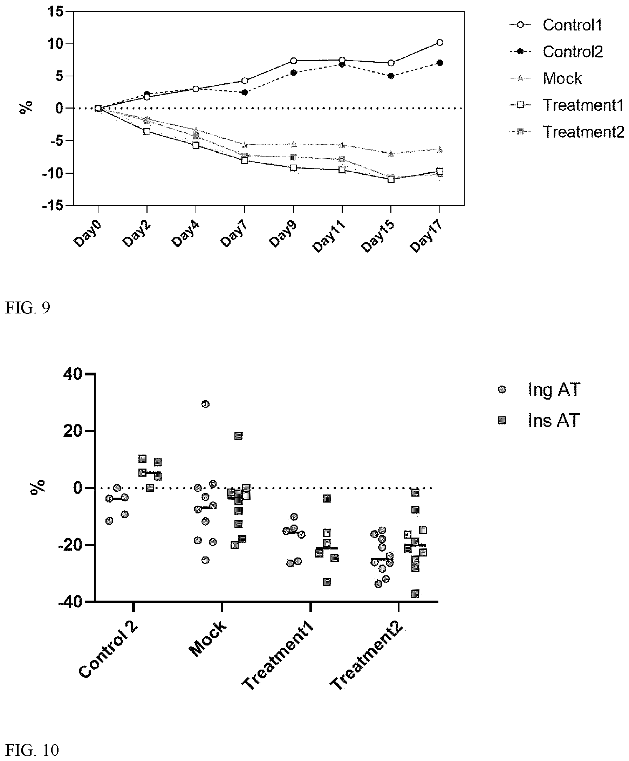

FIG.8 and FIG.9 show the results after administering a DNA vaccine for YFV. Figure 8 measures the change in body weight before and after administration, based on the body weight just before administering the YFV genetic material (DNA). Figure 9 illustrates the weight reduction effects on inguinal adipose tissue (Ing AT) and interscapular adipose tissue (Ins AT) due to the treatment containing YFV genetic material (DNA).

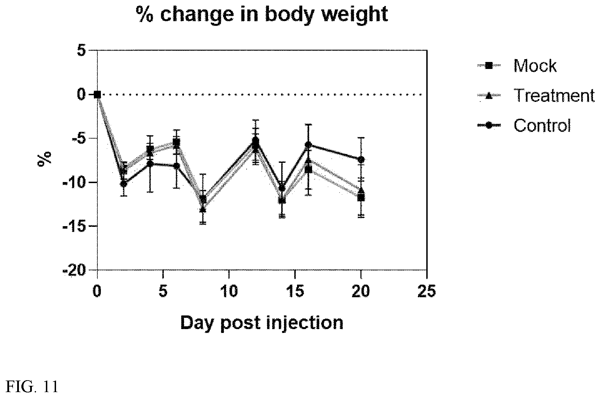

FIG. 10 and FIG. 11 show the results after administering a DNA vaccine for VZV. Figure 10 measures the change in body weight before and after administration, based on the body weight just before administering the VZV genetic material (DNA). Figure 11 shows the weight reduction effects on inguinal adipose tissue due to the administration of the vaccine containing VZV genetic material (DNA).

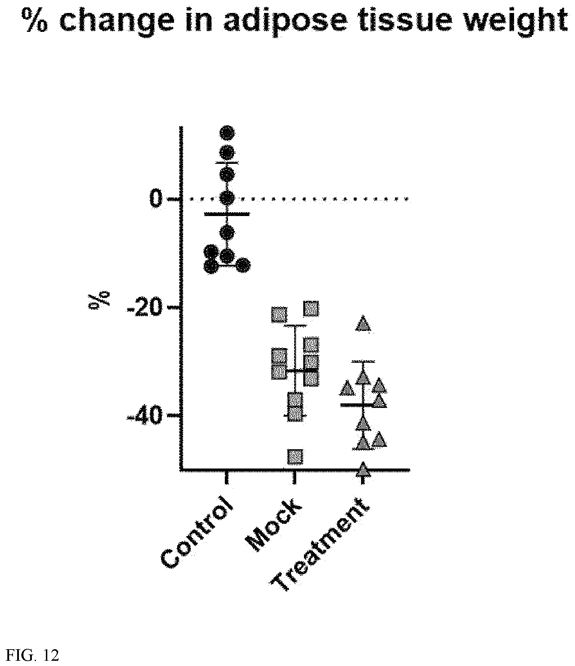

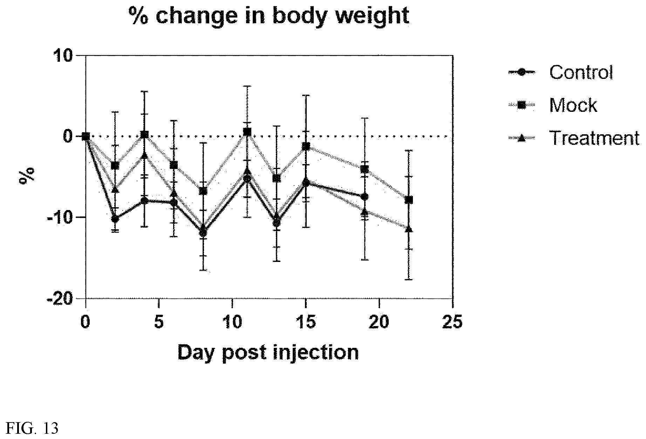

FIG. 12 and FIG. 13 show the results after administering an mRNA vaccine for YFV. Figure 12 measures the change in body weight before and after administration, based on the body weight just before administering the YFV genetic material (mRNA). Figure 13 illustrates the weight reduction effects on inguinal adipose tissue due to the treatment containing YFV genetic material (mRNA).

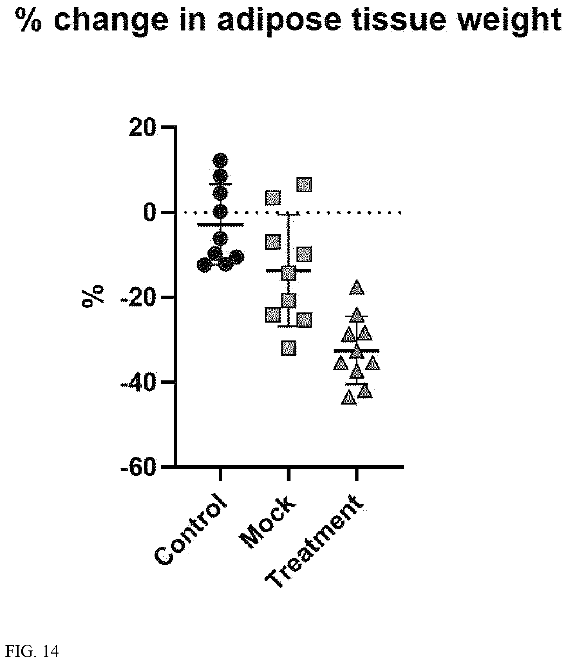

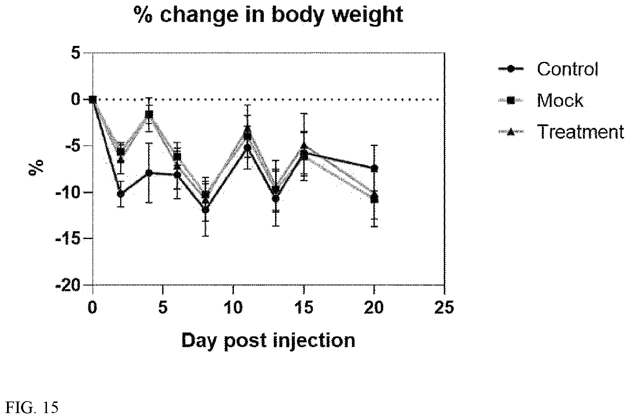

FIG. 14 and FIG. 15 show the results after administering an mRNA vaccine for VZV. Figure 14 measures the change in body weight before and after administration, based on the body weight just before administering the VZV genetic material (mRNA). Figure 15 shows the weight reduction effects on inguinal adipose tissue due to the administration of the vaccine containing VZV genetic material (mRNA).

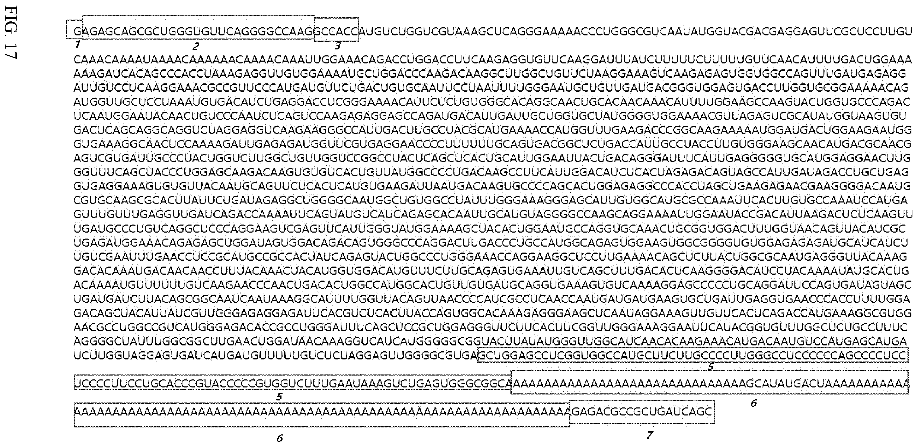

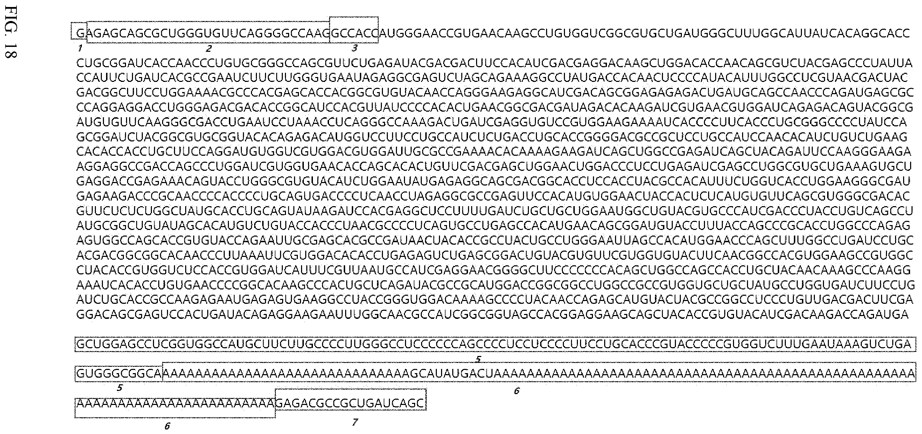

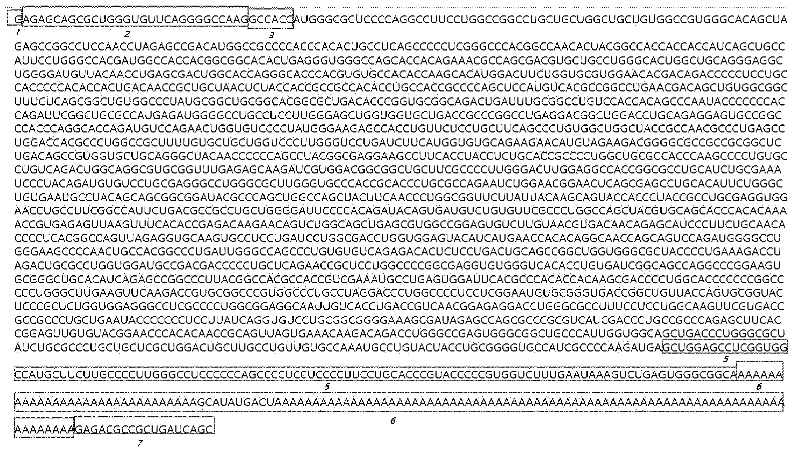

FIGs. 16 to 18 show the mRNA sequences of SEQ ID Nos. 2, 4, and 6, respectively. Here, 1 refers to the capping sequence, 2 refers to the 5'-UTR sequence, 3 refers to the Kozak sequence, 5 refers to the 3'-UTR sequence, 6 refers to the poly A sequence, and 7 refers to the primer sequence. The unmarked section (4) represents the GOI (Gene Of Interest) sequence. Figure 16 includes the gene encoding the structural protein of the yellow fever virus, Figure 17 includes the gene for the gE protein of the varicella-zoster virus, and Figure 18 includes the gene encoding the E2-E1 protein of RuV.

### [MODE FOR INVENTION]

Hereinafter, in order to help understanding of the present invention, it will be described in detail by examples and the like. However, the examples according to the present invention may be modified in many different forms, and the scope of the present invention should not be construed as being limited to the following examples. The examples of the present invention are provided to explain the present invention more completely to those skilled in the art. All sequences are described from the 5' to the 3' direction unless otherwise specified.

### 1. Preparation of vaccine (treatment) for degrading adipocytes

For the experiment, genetic material to be administered to individuals with pre-existing immunity and an immunoadjuvant that can be administered together with the genetic material were prepared as follows:

### (1) Preparation of foreign antigens

1) Preparation of genetic material for the yellow fever virus (Yellow fever virus (strain: 17D); YFV) First, the genetic material coding for structural proteins (capsid protein, prM protein, envelope protein, from 119 to 2452 (2,334 bp)) was prepared from the YFV genome (GenBank: X03700.1) (SEQ. ID NOs. 1 and 2).
2) Preparation of genetic material for the varicella-zoster virus (Varicella zoster virus (strain: Oka); VZV)
   The genetic material coding for the gE protein (1,872 bp) was prepared from the VZV genome (SEQ. ID NOs. 3 and 4).
3) Preparation of genetic material for the rubella virus (Rubella virus (strain: RA27/3); RuV)
   The genetic material coding for the E2-E1 protein (2,358 bp) was prepared from the RuV genome (SEQ. ID NOs. 5 and 6).
4) Preparation of genetic material coding for firefly luciferase (FLuc) (SEQ. ID NO. 15)

### (2) Preparation of vectors (DNA vaccine treatments) expressed in animal cells

The genetic material of YFV was inserted into the gWiz^{™} vector, which allows protein expression in animal cells. A Kozak sequence (GCCACC) was added at the front of the gene, and a stop codon was added at the end of the gene. The genetic material of VZV was inserted into the gWiz^{™} vector, with a Kozak sequence added at the front of the gene. The genetic material of RuV was inserted into the gWiz^{™} vector, with a Kozak sequence (GCCACC) and a start codon (ATG) added at the front of the gene.

The vector map is shown in Figure 1. The genes were inserted at the restriction enzyme sites of SalI and NotI.

The cloned YFV-antigen plasmid (vector backbone: gWiz^{™}), VZV-antigen plasmid (vector backbone: gWiz^{™}), and RuV-antigen plasmid (vector backbone: gWiz^{™}) were transformed into DH5α competent cells. The cells were then cultured in bulk, and the plasmids were recovered using a plasmid prep kit.

### (3) Preparation of auxiliary genetic material

Next, an immunoadjuvant was prepared as an auxiliary genetic material. The gene sequences (SEQ ID NOs. 7 and 8) of the coding region (p35; 127-774 (648 bp), p40; 35-1042 (1,008 bp)) of p35 (GenBank: M86672.1) and p40 (GenBank: M86671.1) of IL-12 were inserted into the pSF-CMV-CMV-Sbfl vector along with the kozak sequence. P35 was inserted into the EcoRI, XhoI sites, and p40 was inserted into the SalI, SpeI sites. The vector map is shown in Fig. 2.

The cloned IL-12 plasmid (vector backbone: pSF-CMV-CMV-Sbfl) was transformed into DH5a competent cells. The cells were mass-cultured, and the plasmids were recovered using a plasmid prep kit.

### (4) Preparation of vector for in vitro transcription (IVT)

The genetic material of YFV was inserted between the T7 promoter sequence and the bGH poly(A) signal sequence of pcDNA^{™}3.1(+) through cloning. The 5' untranslated region sequence and Kozak sequence of the Homo sapiens PPARG-related coactivator 1 (PPRC1) gene were added in front of the gene, and the stop codon (TGA), the 3' untranslated region sequence of the Homo sapiens alpha-1-globin gene, the poly A sequence, and the Esp3i enzyme cut site sequence were added at the end of the gene. The vector map is shown in Fig. 3.

The genetic material of VZV was inserted between the T7 promoter sequence and the bGH poly(A) signal sequence of pcDNA3.1^{™}(+) through cloning. The 5' untranslated region sequence and Kozak sequence of the Homo sapiens PPARG-related coactivator 1 (PPRC1) gene were added in front of the gene, and the 3' untranslated region sequence, poly A sequence, and Esp3i enzyme cut site sequence of the Homo sapiens alpha-1-globin gene were added at the end of the gene.

The genetic material of RuV was inserted between the T7 promoter sequence and the bGH poly(A) signal sequence of pcDNA3.1^{™}(+) through cloning. The 5' untranslated region sequence, Kozak sequence, and start codon of the Homo sapiens PPARG-related coactivator 1 (PPRC1) gene were added in front of the gene, and the 3' untranslated region sequence, poly A sequence, and Esp3i enzyme cut site sequence of the Homo sapiens alpha-1-globin gene were added at the end of the gene.

The genetic material encoding FLuc was inserted between the T7 promoter sequence and the bGH poly(A) signal sequence of pcDNA^{™}3.1(+) through cloning. The 5' untranslated region sequence and Kozak sequence of the Homo sapiens PPARG-related coactivator 1 (PPRC1) gene were added in front of the gene, and the stop codon, the 3' untranslated region sequence of the Homo sapiens alpha-1-globin gene, the poly A sequence, and the Esp3i enzyme cut site sequence were added at the end of the gene.

The cloned YFV-antigen plasmid (vector backbone: pcDNA^{™}3.1(+)), VZV-antigen plasmid (vector backbone: pcDNA^{™}3.1(+)), RuV-antigen plasmid (vector backbone: pcDNA^{™}3.1(+)), and FLuc plasmid (vector backbone: pcDNA^{™}3.1(+)) were transformed into DH5α competent cells. The cells were mass-cultured, and the plasmids were recovered using a plasmid prep kit.

### (5) Preparation of genetic material (mRNA vaccine therapeutics) expressed in animal cells through IVT

YFV-antigen plasmid (vector backbone: pcDNA3.1^{™}(+)), VZV-antigen plasmid (vector backbone: pcDNA^{™}3.1(+)), and RuV-antigen plasmid (vector backbone: pcDNA^{™}3.1(+)) were used as templates for PCR to prepare linearized DNA (SEQ ID NO: 9, SEQ ID NO: 10, and SEQ ID NO: 11). Primer information is shown in Table 1 below.

**Table 1.**

| Direction | oligo name | Sequence No. | Remarks |
|---|---|---|---|
| F | F_T7_M4AG | SEQ ID NO. 17 | Primers for the PCR (linearized DNA preparation) (Designed and used a universal primer that can be used regardless of the type of GOI) |
| R | R_esp3i+11bp | SEQ ID NO. 18 | |

Using the FLuc plasmid (vector backbone: pcDNA^{™}3.1(+)) as a template, PCR was performed to induce a frameshift 12 bp downstream of the start codon, and linearized DNA with a stop codon was prepared (SEQ ID NO. 15). The primer information is shown in Table 2 below.

**Table 2.**

| Directi on | oligo name | Sequence No. | Remarks |
|---|---|---|---|
| F | f_fluc | SEQ ID NO. 19 | PCR was performed in two steps to induce a stop codon in Flu |
| R | r_fluc_2 | SEQ ID NO. 20 | |
| F | F_t7_fluc_shift | SEQ ID NO. 21 | 1. 1st PCR - template: FLuc plasmid (vector backbone: pcDNA^{™}3.1(+); SEQ ID NO. 15) - primers: (F) f_fluc, (R) r_fluc_2 |
| | | | 2. 2nd PCR - template: amplicon of 1st PCR - primers: (F) F_t7, flue shift, (R) r_fluc2 |

IVT was performed using linearized DNA as a template. Following this, DNase treatment was conducted, and mRNA was precipitated and purified using LiCl. The prepared mRNA was transfected into the Vero E6 cell line to confirm antigen expression. It was confirmed that the FLuc mRNA containing the stop codon did not result in protein expression before it was used.

### 2. Confirmation of death of adipocytes

### (1) Experimental method

### 1) Diet induced obese (DIO) mouse production

C57BL/6J, 3w, female mice were fed a normal diet and a high fat diet with 60% kcal from fat, respectively. Body weight was measured at weekly intervals, and the animals were reared for 10 to 15 weeks to check weight gain before use in the experiment.

C57BL/6J, 4w, male mice were fed a high fat diet with 60% kcal from fat. Body weight was measured at weekly intervals, and the animals were reared for 10 to 15 weeks to check weight gain before use in the experiment.

### 2) Induction of pre-existing immunity and measurement of antibody titer

Pre-existing immune response was induced using attenuated live viruses as vaccine strains. YFV (strain: 17D, GenBank: X03700.1) and VZV (strain: Oka, Sky Zoster strain (SK Bioscience)) were used as the vaccine strains.
**(i) YFV immunization:** The YFV vaccine strains (attenuated live virus) was injected into the muscle of the left hind limb of the mouse at a concentration of 2E5 pfu/time 3 times at a 2-week interval. Blood was collected 2 weeks after the last injection, and serum was separated.

The confirmation of Antibody titer: In the separated serum, antibodies specific to YFV were detected through ELISA analysis. Through ELISA analysis, YFV used for injection was coated on a plate at a concentration of 5E4 pfu/well, and an analysis sample (serum) was diluted and reacted for 2h. After washing, an anti-mouse IgG-HRP secondary antibody was diluted by 1/4000 and treated, and then reacted for 2h. After washing, a color forming solution was added and reacted for 10 min to measure an O.D value.

Results: Normal immune induction (antibody response) occurred by YFV vaccine administration.

The results are shown in Fig. 4. Compared to the negative control (NC) group administered with medium, an increase in YFV-specific antibodies was confirmed in the YFV-ND and YFV-HFD groups administered with YFV.

YFV-specific pre-existing immunity induction for treatment administration was confirmed.

**(ii) YFV immunization (2):** YFV (attenuated live virus) was administered twice to the left hind limb muscle of the mouse at 2-day intervals 3 weeks before starting treatment administration. (Intramuscular injection, 1^{st} shot: 2E4 pfu/injection, 2^{nd} shot: 4E4 pfu/injection) An additional YFV immunization was administered 2-3 days before treatment administration, i.e. 3 weeks after the 1st shot. (Intramuscular injection, 3^{rd} shot: 1E5 pfu/injection)

The confirmation of Antibody titer: Blood samples were collected before YFV administration and 3 weeks after the first shot administration, and serum was separated. For ELISA analysis, YFV used in the injection was coated on the plate at a concentration of 1E4 pfu/well, blocked with 2% skim milk, and then diluted serum was reacted with the antigen for 1 h. After washing, anti-mouse IgG-HRP secondary antibody was diluted 1/1000 and reacted for 1 h. After washing, the color development solution was added and reacted for 10 min, and the OD value was measured.

Results: Normal immune induction (antibody response) occurred by administration of YFV vaccine strain. The results are shown in Fig. 5. Compared with serum before YFV immunization (pre-vaccination), an increase in YFV-specific antibody was confirmed in serum sampled after two YFV immunizations (post-vaccination). Induction of YFV-specific pre-existing immunity for therapeutic administration was confirmed.

**(iii) VZV immunization:** Mice were immunized twice with VZV (attenuated live virus) at 4-week intervals. (Subcutaneous injection, 1^{st} shot: 9E3 pfu/injection, 2^{nd} shot: 9E3 pfu/injection) An additional VZV immunization was performed one week before the administration of treatment. (Subcutaneous injection, 3^{rd} shot: 9E3 pfu/injection)

The confirmation of Antibody titer: Blood was collected before VZV administration and 3 weeks after the 2^{nd} shot administration, and serum was separated. The separated serum was analyzed by ELISA to detect VZV gE protein-specific antibodies. For ELISA analysis, recombinant VZV gE protein was coated on the plate at a concentration of 100 ng/well, blocked with 2% skim milk, and then the diluted serum was reacted with the antigen for 1 h. After washing, anti-mouse IgG-HRP secondary antibody was diluted 1/1000 and reacted for 1 h. After washing, the color development solution was added and reacted for 10 min to measure the OD value.

Results: Normal immunity (antibody response) occurred by VZV vaccine administration. The results are shown in Fig. 6 and Fig. 7. Compared to the serum before VZV immunization (pre-vaccination), an increase in VZV gE protein-specific antibodies was confirmed in the serum sampled after two VZV immunizations (post-vaccination). Induction of VZV-specific pre-existing immunity for therapeutic administration was confirmed.

**(iv) RuV immunization:** The experimental animals were immunized 2-3 times with the MMR vaccine (vaccine containing RuV attenuated live virus) at 4-week intervals. (Subcutaneous injection)

The confirmation of Antibody titer: Blood samples were collected before MMR vaccination and 3 weeks after the second vaccination, and serum was isolated. The separated serum was detected for RuV E1 protein-specific antibodies through ELISA analysis. For ELISA analysis, recombinant RuV E1 protein was coated on the plate at a concentration of 100 ng/well, blocked with 2% skim milk, and the diluted serum was reacted with the antigen for 1 h. After washing, anti-mouse IgG-HRP secondary antibody was diluted 1/1000 and reacted for 1 h. After washing, the color development solution was added and reacted for 10 min, and the OD value was measured.

Results: Similar to YFV and VZV administration, induction of RuV-specific pre-existing immunity for therapeutic administration was confirmed.

### 3) Confirmation of therapeutic vaccine administration and adipose tissue reduction effect

### (i) DNA vaccine administration (1) (YFV-immunized group)

Method: Normal mice or mice induced to become obese were divided into groups administered YFV vaccine and groups not administered YFV vaccine, respectively, and used in the experiment. All mice were provided with a normal diet (ND) starting 3 days before the therapeutic vaccine administration. The DNA vaccine (therapeutic agent) was administered to the inguinal (scapula) adipose tissue and inguinal (groin) adipose tissue of the mice. The treatment was administered 5 times at 2 sites on the left or right side of each tissue at 2-3 day intervals. The treatment was administered at a concentration of 100 µg/tissue of YFV-antigen-encoding DNA and 50 µg/tissue of II,-12-encoding DNA, for a total of 150 µg/time, and an empty vector of the same amount as the total amount of DNA vaccine administered was administered to the mock group.

Body weights were measured before and after the therapeutic vaccine administration. The mice were necropsied 7 days after the last therapeutic vaccine administration. Left/right inguinal adipose tissue and interscapular adipose tissue were separated and their respective weights were measured.

**Table 3.**

| Group | Classific ation | Diet 1 | Diet 2(3 days before treatment ~ autopsy) | Pre-existing immunity induction (3th) | Information of treatment | Number of treatment doses | Number of subjects |
|---|---|---|---|---|---|---|---|
| Control 1 | Normal | ND | ND | Medium | - | - | 5 |
| Control 2 | | | | YFV vaccine strain | - | - | 5 |
| Mock | DIO | HFD | | YFV vaccine strain | Empty vector | 5 times at 2-day intervals | 10 |
| Treatment group 1 | | | | YFV vaccine strain | YFV-antigen DNA | 5 times at 2-day intervals | 10 |
| Treatment group 2 | | | | YFV vaccine strain | YFV-antigen DNA, IL-12 DNA | 5 times at 2-day intervals | 10 |

| | | | | | | | |
|---|---|---|---|---|---|---|---|
| (DIO: Diet-induced obesity, ND: Normal diet, HFD: High-fat diet; YFV-antigen DNA: SEQ ID NO: 1, IL-12 DNA: combination of SEQ ID NOs: 5 and 6) | | | | | | | |

Results: The average body weight change rate and adipose tissue reduction effect after treatment administration for each group in Table 3 above were confirmed and shown in Figures 8 and 9.

Referring to Figure 8, body weight was measured before and after treatment administration, and the body weight change was measured based on the body weight immediately before administration. In the case of the group that was not treated at all (control 1), body weight gradually increased, but the Mock, treatment 1, and treatment 2 groups all showed a body weight reduction effect, and the treatment 1 and treatment 2 groups showed a greater decrease compared to the Mock group. The treatment 1 group also showed an excellent body weight reduction effect, and the treatment 2 group, which was administered with IL-12, showed the greatest body weight reduction. These results show that treatment 1 alone showed an excellent body weight reduction effect.

Figure 9 shows the fat weight reduction effect by treatment administration. The treatment was administered to only the left or right side, and on the 7th day after 5 administrations of the treatment, the inguinal AT (adipose tissue) and interscapular AT on the left and right sides of the mice were separated and the AT weight was measured. The results of Figure 9 are presented by group by calculating the effect of reducing fat weight by comparing the left and right AT weights within the same individual. When comparing the control group (no treatment administered) and the mock group (empty vector administered), it can be seen that the adipose tissue weight was reduced by administering treatment 1 and treatment 2.

These results show that the treatment of the present invention is not only effective in reducing body weight, but also has the effect of reducing the weight of adipose tissue. Compared to the mock group, the average value of the adipose tissue weight was significantly reduced. It can be seen that the change in adipose tissue weight was much greater in the treatment 2 administration group that was administered together with IL-12.

### (ii) Administration of DNA Vaccine (2) (VZV-immunized group)

Method: Among mice induced to be obese by a high-fat diet (HFD), those confirmed to have pre-existing VZV-specific immunity were selected for the administration of the DNA vaccine (therapeutic agent). Mice were fed HFD until the administration of the therapeutic agent, and from the day of administration, they were alternately fed normal diet (ND) and HFD every three days.

The treatment was administered to the inguinal adipose tissue of the mice. The agent was injected at four sites on either the left or right side of each tissue, with a total of five administrations at two-day intervals. The treatment was administered at a concentration of 100 µg/tissue of VZV-antigen-encoding DNA, totaling 100 µg per administration, and an equal amount of empty vector was administered to the mock group.

Body weights were measured before and after therapeutic vaccine administration. Mice were necropsied 12-14 days after the last therapeutic vaccine administration. The left and right inguinal adipose tissues were isolated, and their weights were measured.

**Table 4.**

| Group | Classi ficatio n | Diet 1 | Diet 2(Day of treatment administration -Autopsy) | Pre-existing immunity induction (3th) | Information of treatment | Number of treatment doses | Number of times treatment is administered | Number of subjects |
|---|---|---|---|---|---|---|---|---|
| Control | DIO | HFD | Feed ND and HFD alternately every 3 days | VZV vaccine strain | - | - | - | 9 |
| Mock | | | | VZV vaccine strain | Empty vector | 100 µg/tissue | 5 times at 2-day intervals | 10 |
| Treatment | | | | VZV vaccine strain | VZV-antigen DNA | 100 µg/tissue | 5 times at 2-day intervals | 9 |

| | | | | | | | | |
|---|---|---|---|---|---|---|---|---|
| (DIO: Diet-induced obesity, ND: Normal diet, HFD: High-fat diet; VZV-antigen DNA: SEQ ID No. 3) | | | | | | | | |

Results: The average body weight change rate and adipose tissue reduction effect after treatment administration for each group in Table 4 above were confirmed, respectively, and are shown in Figures 10 and 11.

Referring to Figure 10, body weight was measured before and after treatment administration, and the body weight change rate was calculated based on the body weight immediately before administration. Weight loss was observed in all groups that were alternately fed ND and HFD for 3 days from the day of treatment administration. In particular, a greater weight loss was confirmed in the mock and treatment groups that were administered DNA compared to the control group (no treatment administered). The weight loss rates before administration (day 0) and immediately before autopsy in the mock and treatment groups were similar.

Figure 11 shows the fat weight reduction effect by treatment administration. The treatment was administered to only the left or right side, and the weight of the left and right inguinal adipose tissues of the mice was measured on days 12 to 14 after 5 administrations of the treatment. The results in Fig. 11 are presented by group, calculating the rate of adipose tissue reduction by comparing the weight of left and right adipose tissues separated from the same individual. A decrease in adipose tissue was confirmed in the mock group and treatment group that received DNA compared to the control group (no treatment administered). In particular, a greater rate of reduction was confirmed in the treatment group compared to the mock group.

**Table 5**

| | Control | Mock | Treatment |
|---|---|---|---|
| 1 | 4.6 | -31.9 | -44.3 |
| 2 | 12.3 | -28.9 | -41.3 |
| 3 | 8.6 | -20.2 | -32.9 |
| 4 | -12.4 | -26.9 | -34.3 |
| 5 | -6.1 | -21.4 | -23.0 |
| 6 | 0.2 | -47.5 | -37.2 |
| 7 | -10.5 | -33.2 | -44.9 |
| 8 | -9.7 | -30.1 | -34.9 |
| 9 | -12.2 | -39.6 | -49.8 |
| 10 | - | -37.1 | - |
| **(mean)** | **-2.80** | **-31.69** | **-38.05** |
| **(SD)** | **9.47** | **8.26** | **8.02** |

As can be seen in Table 5 and Figure 11, the average value of adipose tissue reduction in the treatment group of the present invention significantly decreased compared to the Control group. Furthermore, when compared to the Mock group, the degree of adipose tissue reduction was even more impressive. Considering the weight and size of the adipose tissue, the achievement of such a reduction effect is a remarkable outcome.

### (iii) Administration of mRNA Vaccine (3) (YFV-immunized group)

Method: Among mice induced with obesity through a high-fat diet (HFD), those confirmed to have YFV-specific pre-existing immunity were selected for mRNA vaccine (treatment) administration. Mice were fed HFD until the administration of the treatment, and from the day of treatment, they were alternately fed normal diet (ND) and HFD every three days. The treatment was administered to the inguinal adipose tissue of the mice. The treatment was given in four spots on either the left or right side of each tissue, at intervals of two days, for a total of five administrations. The treatment was administered at a concentration of 10 µg/tissue of YFV-antigen-encoding mRNA per session. The mock group received the same total amount of mRNA as the treatment group, consisting of FLuc-encoding mRNA that does not express protein due to the inclusion of a stop codon. Body weight was measured before and after the administration of the therapeutic vaccine. After 12 to 14 days following the last administration of the therapeutic vaccine, the mice were euthanized, and the left and right inguinal adipose tissues were separated and weighed.

**Table 6**

| Group | Class ificat ion | Diet 1 | Diet 2(Day of treatment administration-A utopsy) | P Pre-existing immunity induction (3th) | Information of treatment | Number of treatment doses | Number of times treatment is administere d | Number of subjects |
|---|---|---|---|---|---|---|---|---|
| Control | DIO | HFD | Feed ND and HFD alternately every 3 days | VZV vaccine strain | - | - | - | 9 |
| Mock | | | | YFV vaccine strain | Flue mRNA(with stop codon) | 10 µg/tissue | 5 times at 2-day intervals | 9 |
| Treatmen t | | | | YFV vaccine strain | YFV-antigen mRNA | 10 µg/tissue | 5 times at 2-day intervals | 10 |

| | | | | | | | | |
|---|---|---|---|---|---|---|---|---|
| (DIO: Diet-induced obesity, ND: Normal diet, HFD: High-fat diet; treatment: SEQ ID No.2) | | | | | | | | |

Results: The average change rate in body weight and the effect of adipose tissue reduction after treatment administration for each group, as shown in Table 6, are illustrated in Figures 12 and 13. Referring to Figure 12, body weight was measured before and after the treatment, and the change rate in body weight was calculated based on the weight just before administration. Weight loss was observed in all groups that alternated between normal diet (ND) and high-fat diet (HFD) every three days from the day of treatment administration. Notably, compared to the control group (no treatment), a greater weight loss was confirmed in the mock and treatment (therapeutic) groups that received the mRNA. In particular, the weight loss rate just before euthanasia after treatment was higher in the treatment group compared to the mock group.

**Table 7**

| (%) | Day 0 | Day 2 | Day 4 | Day 6 | Day 8 | Day 11 | Day 13 | Day 15 | Day 19 | Day 22 |
|---|---|---|---|---|---|---|---|---|---|---|
| control | 0.00 | -10.17 | -7.90 | -8.13 | -11.91 | -5.20 | -10.68 | -5.75 | -7.40 | - |
| mock | 0.00 | -3.56 | 0.23 | -3.50 | -6.71 | 0.57 | -5.15 | -1.22 | -4.02 | -7.81 |
| treatment | 0.00 | -6.46 | -2.23 | -6.91 | -11.06 | -4.15 | -9.67 | -5.29 | -9.17 | -11.30 |

Figure 13 illustrates the effect of treatment administration on fat weight reduction. The treatment was administered only to one side (left or right), and after five administrations of the treatment, the left and right inguinal (subcutaneous) adipose tissues of the mice were separated and weighed on days 12 to 14. The results in Figure 13 show the adipose tissue reduction rate calculated by comparing the weights of the left and right adipose tissues separated from the same individual, presented by group. Adipose tissue reduction was observed in the mock group and treatment (therapeutic) group that received the mRNA compared to the control group (no treatment). Notably, a greater reduction rate was confirmed in the treatment group compared to the mock group. The results are presented in Table 8.

**Table 8**

| | Control | Mock | Treatment |
|---|---|---|---|
| 1 | 4.6 | 6.6 | -35.4 |
| 2 | 12.3 | -7.0 | -28.7 |
| 3 | 8.6 | -9.9 | -17.6 |
| 4 | -12.4 | 3.4 | -24.1 |
| 5 | -6.1 | -31.9 | -28.3 |
| 6 | 0.2 | -14.3 | -35.4 |
| 7 | -10.5 | -24.1 | -41.9 |
| 8 | -9.7 | -20.8 | -43.5 |
| 9 | -12.2 | -25.4 | -32.6 |
| 10 | - | - | -37.3 |
| **(mean)** | **-2.80** | **-13.7** | **-32.5** |
| **(SD)** | **9.47** | **13.2** | **8.0** |

### (iv) Administration of mRNA Vaccine (4) (VZV-immunized group)

Method: Among mice induced to be obese by a high-fat diet (HFD), those confirmed to have pre-existing VZV-specific immunity were selected for the administration of the mRNA vaccine (therapeutic agent). Mice were fed HFD until the administration of the therapeutic agent, and from the day of administration, they were alternately fed normal diet (ND) and HFD every three days.

The treatment was administered to the inguinal adipose tissue of the mice. The agent was injected at four sites on either the left or right side of each tissue, with a total of five administrations at two-day intervals. The treatment was administered at a concentration of 10 µg/tissue of VZV-antigen-encoding mRNA per administration. The FLuc-encoding mRNA containing a stop codon, which does not express proteins, was administered to the mock group in an amount equal to the total amount of mRNA in the administered therapeutic agent.

Body weight was measured before and after the administration of the therapeutic vaccine. After 12 to 14 days following the last administration of the therapeutic vaccine, the mice were euthanized. The left and right inguinal adipose tissues were isolated, and their weights were measured.

**Table 9**

| Group | Classification | Diet 1 | Diet 2(Day of treatment administration-A utopsy) | P Pre-existing immunity induction (3th) | Information of treatment | Number of treatment doses | Number of times treatment is administered | Number of subject s |
|---|---|---|---|---|---|---|---|---|
| | | | | | | | | |
| Control | DIO | HFD | Feed ND and HFD alternately every 3 days | VZV vaccine strain | - | - | - | 9 |
| Mock | | | | VZV vaccine strain | Flue mRNA(with stop codon) | 10 µg/tissue | 5 times at 2-day intervals | 10 |
| Treatmen t | | | | VZV vaccine strain | VZV-antigen mRNA | 10 µg/tissue | 5 times at 2-day intervals | 9 |

| | | | | | | | | |
|---|---|---|---|---|---|---|---|---|
| (DIO: Diet-induced obesity, ND: Normal diet, HFD: High-fat diet; VZV-antigen mRNA: SEQ ID No. 4) | | | | | | | | |

Results: The average weight change rates and the effects of adipose tissue reduction for each group after the administration of the therapeutic agent, as shown in Table 9, were confirmed and are presented in Figures 14 and 15.

Referring to Figure 14, body weight was measured before and after the administration of the therapeutic agent, and the weight change rate was calculated based on the weight immediately prior to administration. Weight loss was observed in all groups that were alternately fed normal diet (ND) and high-fat diet (HFD) every three days from the day of administration. Notably, greater weight loss was confirmed in the mock and treatment (therapeutic agent) groups that received mRNA compared to the control group (no treatment). The weight loss rates for the mock and treatment groups before administration (day 0) and just prior to euthanasia after administration were similar. The values are presented in Table 10.

**Table 10**

| (%) | Day 0 | Day 2 | Day 4 | Day 6 | Day 8 | Day 11 | Day 13 | Day 15 | Day 20 |
|---|---|---|---|---|---|---|---|---|---|
| control | 0 | -10.17 | -7.90 | -8.13 | -11.91 | -5.20 | -10.68 | -5.75 | -7.40 |
| mock | 0 | -5.63 | -1.61 | -6.18 | -10.24 | -4.00 | -9.74 | -6.15 | -10.75 |
| treatment | 0 | -6.44 | -1.65 | -7.14 | -10.77 | -3.09 | -9.36 | -4.89 | -10.09 |

Figure 15 shows the effect of weight reduction in adipose tissue due to the administration of the therapeutic agent. The treatment was administered only to either the left or right side, and on days 12 to 14 after five administrations of the therapeutic agent, the left and right inguinal (groin) adipose tissues of the mice were isolated and weighed. The results in Figure 15 present the adipose tissue reduction rates calculated by comparing the weights of the left and right adipose tissues isolated from the same individuals. A reduction in adipose tissue was confirmed in the mock and treatment (therapeutic agent) groups that received mRNA compared to the control group (no treatment). Notably, a greater reduction rate was observed in the treatment group compared to the mock group.

### (v) Administration of DNA Vaccine (5) (MMR-immunized group)

Method: Among mice induced to be obese by a high-fat diet (HFD), those confirmed to have pre-existing RuV-specific immunity were selected for the administration of the mRNA vaccine (therapeutic agent). Mice were fed HFD until the administration of the therapeutic agent, and from the day of administration, they were alternately fed normal diet (ND) and HFD every three days.

The treatment was administered to the inguinal adipose tissue of the mice. The agent was injected at four sites on either the left or right side of each tissue, with a total of five administrations at two-day intervals. The treatment was administered at a concentration of 100 µg/tissue of RuV-antigen-encoding DNA, totaling 100 µg per administration, and an equal amount of empty vector was administered to the mock group.

Body weight was measured before and after the administration of the therapeutic vaccine. After 12 to 14 days following the last administration of the therapeutic vaccine, the mice were euthanized. The left and right inguinal adipose tissues were isolated, and their weights were measured.

Results: Similar to the YFV and VZV treatment groups, weight loss and adipose tissue reduction effects were confirmed in the treatment administration group.

### (vi) Administration of mRNA Vaccine (6) (MMR-immunized group)

Method: Among mice induced to be obese by a high-fat diet (HFD), those confirmed to have pre-existing RuV-specific immunity were selected for the administration of the mRNA vaccine (therapeutic agent). Mice were fed HFD until the administration of the therapeutic agent, and from the day of administration, they were alternately fed normal diet (ND) and HFD every three days.

The treatment was administered to the inguinal adipose tissue of the mice. The agent was injected at four sites on either the left or right side of each tissue, with a total of five administrations at two-day intervals. The treatment was administered at a concentration of 10 µg/tissue of RuV-antigen-encoding mRNA per administration. The FLuc-encoding mRNA containing a stop codon, which does not express proteins, was administered to the mock group in an amount equal to the total amount of mRNA in the administered therapeutic agent.

Body weight was measured before and after the administration of the therapeutic vaccine. After 12 to 14 days following the last administration of the therapeutic vaccine, the mice were euthanized. The left and right inguinal adipose tissues were isolated, and their weights were measured.

Results: Similar to the YFV and VZV treatment experiments, weight loss and adipose tissue reduction effects were confirmed in the treatment administration group.

**Table 11.**

| Sequence information | | |
|---|---|---|
| No. | Explanation | Sequence Number for internal reference and notes |
| SEQ. ID No. 1 | DNA sequence encoding the structural protein of YFV (positions 119-2452 (2,334 bp)) | Reference sequence no. 1a |
| | | DNA sequence encoding the structural protein of YFV (capsid protein, prM protein, envelope protein, 119 ~ 2452 (2,334 bp)) |
| SEQ. ID No. 2 | mRNA sequence encoding the structural protein of YFV (positions 119 ~ 2452 (2,334 bp)) | Reference sequence no. 7b |
| | | YFV-antigen-expressing mRNA prepared by IVT using Reference sequence no. 7a as a template |
| SEQ. ID No. 3 | DNA sequence encoding VZV gE protein (1,872 bp) | Reference sequence number 2a |
| | | VZV gE protein (1,872 bp) DNA sequence |
| SEQ. ID No. 4 | mRNA sequence encoding VZV gE protein (1,872 bp) | Reference sequence number 8b |
| | | VZV-antigen-expressing mRNA prepared by IVT using Reference sequence number 8a as a template |
| SEQ. ID No. 5 | DNA sequence encoding the E2-E1 protein of RuV (2,358 bp) | Reference sequence number 3a |
| | | DNA sequence of E2-E1 protein (2,358 bp) of RuV |
| SEQ. ID No. 6 | mRNA sequence encoding the E2-E1 protein of RuV (2,358 bp) | Reference sequence number 9b |
| | | RuV-antigen-expressing mRNA prepared by IVT using Reference sequence number 9a as a template |
| SEQ. ID No. 7 | DNA sequence of p35 of IL-12 | Reference sequence number 5 |
| | | IL-12 p35 (GenBank: M86672.1; 127-774 (648 bp)) |
| SEQ. ID No. 8 | DNA sequence of p40 of IL-12 | Reference sequence number 6 |
| | | IL-12 p40 (GenBank: M86671.1; 35-1042 (1,008 bp)) |
| SEQ. ID No. 9 | Template DNA of sequence number 2 | Reference sequence number 7a |
| | | Linearized DNA prepared by performing PCR using YFV-antigen plasmid (vector backbone: pcDNA3.1^{™}(+)) as a template |
| SEQ. ID No. 10 | Template DNA of sequence number 4 | Reference sequence number 8a |
| | | Linearized DNA prepared by performing PCR using VZV-antigen plasmid (vector backbone: pcDNA3.1^{™}(+)) as a template |
| SEQ. ID No. 11 | Template DNA of sequence number 6 | Reference sequence number 9a |
| | | Linearized DNA prepared by performing PCR using RuV-antigen plasmid (vector backbone: pcDNA3.1^{™}(+)) as a template |
| SEQ. ID No. 12 | Amino acid sequence of the structural protein of YFV | Reference sequence number 1b |
| | | Structural protein of YFV (capsid protein, prM protein, envelope protein): Amino acid sequence of the expressed protein |
| SEQ. ID | Amino acid sequence of the | Reference sequence number 2b |
| No. 13 | VZV gE protein | VZV gE protein: Amino acid sequence of the expressed protein |
| SEQ. ID No. 14 | Amino acid sequence of the E2-E1 protein of RuV | Reference sequence number 3b |
| | | E2-E1 protein of RuV: Amino acid sequence of the expressed protein |
| SEQ. ID No. 15 | Genetic material encoding firefly luciferase | Reference sequence number 4a |
| | | Firefly luciferase (FLuc) (1,653 bp) |
| SEQ. ID No. 16 | Amino acid sequence of Firefly luciferase | Reference sequence number 4b |
| | | Firefly luciferase (FLuc): Amino acid sequence of expressed protein |
| SEQ. ID No. 17 | F_T7_M4AG primer | |
| SEQ. ID No. 18 | R_esp3i+1 1bp primer | |
| SEQ. ID No. 19 | f_fluc primer | |
| SEQ. ID No. 20 | r_fluc_2 primer | |
| SEQ. ID No. 21 | F_t7_fluc_shift primer | |

### [INDUSTRIAL APPLICABILITY]

The present invention can prevent and/or treat obesity. The present invention provides a method which can prevent and/or treat obesity by injecting into a localized region.

## Claims

1. A pharmaceutical composition for improving, suppressing, or treating obesity or reducing the size or volume of target tissue, comprising at least one virus selected from the group consisting of yellow fever viruses, varicella-zoster virus, and rubella viruses;
a protein derived from said virus; or
a genetic material encoding said virus or protein.

2. The pharmaceutical composition according to claim 1, the pharmaceutical composition is a pharmaceutical composition for reducing the size or volume of a target tissue.

3. The pharmaceutical composition according to claim 1, wherein the genetic material is polynucleotide encoding at least one protein selected from the group consisting of a structural protein of yellow fever virus, glycoprotein E of varicella-zoster virus, and E2 and E1 proteins of rubella virus.

4. The pharmaceutical composition according to claim 3, wherein the polynucleotide is DNA or RNA.

5. The pharmaceutical composition according to claim 1, wherein the genetic material comprises a nucleic acid sequence consisting of a polynucleotide selected from the group consisting of SEQ ID NOs: 1 to 6.

6. The pharmaceutical composition according to claim 1, wherein the pharmaceutical composition further comprises a genetic material encoding a cytokine.

7. The pharmaceutical composition according to claim 6, wherein the cytokine is one or more selected from the group consisting of IL-12, IL-2, IL-4, IL-5, IFN-γ, IL-10, IL-1, IL-6, INF-alpha, INF-beta, TNF-alpha, and TNF-beta.

8. The pharmaceutical composition according to claim 1, wherein the genetic material causes an intracellular immune response.

9. The pharmaceutical composition according to claim 1, wherein the composition locally acts, and
the pharmaceutical composition is applied after at least one antigen for creating an immune environment is first applied to a subject.

10. The pharmaceutical composition according to claim 9, wherein the antigen comprises a virus, protein, or an epitope thereof introduced from outside the human body.

11. The pharmaceutical composition according to claim 1, wherein the genetic material is administered to target tissue orally,
by injection through transdermal, intramuscular, peritoneal, intravenous, subcutaneous or nasal route, or
by electroporation, gene gun, liposome, dendrimers, nanoparticles, or transfer vectors.

12. The pharmaceutical composition according to claim 1, wherein the dose of the genetic material in the composition is 0.1~1,000ug/site per one time inoculation.

13. The pharmaceutical composition according to claim 1, wherein the composition is administered to a subject for reducing or shrinking the size of adipose tissue, or killing of adipose tissue, and
wherein the composition is administered at least once to an area for reducing or shrinking the size of adipose tissue, or killing adipose tissue after confirming the production of antibodies against at least one of the viruses by administering a vaccine for preventing any one or more viruses selected from the group consisting of yellow fever, varicella-zoster virus and rubella viruses.

14. The pharmaceutical composition according to claim 1, wherein the composition is a vaccine.

15. A kit, comprising (a) the pharmaceutical composition according to any one of claims 1 to 14; and
(b) an administration guide for administration of the pharmaceutical composition.

16. The kit according to claim 15,
wherein the kit further comprises (c) a composition comprising an antigen for creating an immune environment prior to administration of the (a) composition.

17. The kit according to claim 15,
wherein the (a) pharmaceutical composition further comprises a genetic material encoding a cytokine.

18. A syringe filled with a vaccine composition for size or volume reduction of a target tissue, comprising at least one virus selected from the group consisting of yellow fever, varicella-zoster virus and rubella viruses; or genetic material encoding a protein derived from thereof.

19. The syringe according to claim 18, wherein the syringe further comprises a genetic material encoding a cytokine.

20. A method for reducing the size or volume of a target tissue of a subject, comprising administering to the target tissue of the subject a genetic material encoding at least one virus selected from the group consisting of yellow fever, varicella-zoster virus and rubella viruses.

21. The method according to claim 20, wherein the method induces reduction or death of the number of adipose cells or the size of adipose tissue of the subject.

22. The method according to claim 20, wherein the method comprises
i) administering to a subject for reducing or shrinking the size of adipose tissue, or killing adipose tissue a vaccine for preventing at least one virus selected from the group consisting of yellow fever, varicella-zoster virus and rubella viruses,
ii) confirming the production of antibodies by the vaccine, and
iii) administering a pharmaceutical composition comprising at least one virus selected from the group consisting of yellow fever, varicella-zoster virus and rubella viruses; a protein derived from the virus; or the genetic material encoding the virus or the protein.

23. The method according to claims 21 or 22, wherein the method further comprises iv) administering at least one cytokine selected from the group consisting of IL-12, IL-2, IL-4, IL-5, IFN-γ, IL-10, IL-1, IL-6, INF-alpha, INF-beta, TNF-alpha, and TNF-beta simultaneously or sequentially with the pharmaceutical composition.

24. Use of composition comprising at least one virus selected from the group consisting of yellow fever, varicella-zoster virus and rubella viruses; a protein derived from the virus; or the genetic material encoding the virus or the protein for the reduction the size or volume of a target tissue, or improvement, suppression, or treatment an obesity.

25. The use according to claim 24, wherein the genetic material comprises a nucleic acid sequence consisting of a polynucleotide selected from the group consisting of SEQ ID NOs: 1 to 6.
